(19) Europäisches Patentamt
European Patent Office
Office européen des brevets

(11) EP 1 170 298 A1

# (12) EUROPÄISCHE PATENTANMELDUNG

(43) Veröffentlichungstag:
**09.01.2002 Patentblatt 2002/02**

(51) Int Cl.$^7$: **C07F 7/08**

(21) Anmeldenummer: **01114733.7**

(22) Anmeldetag: **22.06.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **05.07.2000 DE 10032632**

(71) Anmelder: **Bayer Aktiengesellschaft
51368 Leverkusen (DE)**

(72) Erfinder:
- **Marhold, Albrecht, Dr.
51373 Leverkusen (DE)**
- **Wiedemann, Jürgen, Dr.
51371 Leverkusen (DE)**
- **Bach, Sebastian
86462 Achsheim (DE)**

## (54) Polyfluoralkylsilane

(57) Die Erfindung betrifft Polyfluoralkylsilane, ein Verfahren zu ihrer Herstellung, ihre Verwendung zur Herstellung von Flüssigkristallen, ein Verfahren zur Herstellung von Flüssigkristallen sowie neue Flüssigkristalle.

EP 1 170 298 A1

Printed by Jouve, 75001 PARIS (FR)

## Beschreibung

Die Erfindung betrifft Polyfluoralkylsilane, ein Verfahren zu ihrer Herstellung sowie ihre Verwendung und mit ihnen hergestellte Flüssigkristalle.

Fluorierte organische Moleküle sind von großer Bedeutung für die Herstellung von Polymeren, Agrochemikalien und Pharmazeutika. Vor allem auf dem Gebiet der Pharmazie gelang es, eine Vielzahl von wirksamen antiviralen, antitumoralen und antifungalen Substanzen zu entwickeln, deren Wirksamkeit auf der Einführung einer oder mehrerer polyfluorierter Gruppen basiert.

In der Pharmazie besteht besonderes Interesse an Perfluoralkylverbindungen, da die Anwesenheit von Perfluoralkylgruppen den Transport und die Absorptionsrate eines eingenommenen Medikamentes im Körper begünstigt. Weiterhin finden Perfluoralkylverbindungen bei der Herstellung von Flüssigkristallen ihre Anwendung.

Perfluoralkylsilylverbindungen sind in der Lage, Perfluoralkylgruppen auf andere Moleküle zu übertragen.

Es ist bekannt, Perfluoralkylsilylverbindungen durch Umsetzung von Perfluoralkylbromiden oder -iodiden mit Trimethylchlorsilan herzustellen. Die Umsetzung erfolgt in Gegenwart von Tris(diethylamido)phosphin (Ruppert et al., Tetrahedron Lett. 25, 1984, 2195-2198) oder Tetrakis(dimethylamino)ethan (Pawelke, J. Fluorine Chem. 42, 1989, 429-433). Nachteilig an diesen Verfahren ist, dass Perfluoralkylbromide und -iodide nicht preiswert erhältlich sind. Die hohen Kosten der Ausgangsverbindungen schränken die Anwendbarkeit der oben beschriebenen Verfahren für industrielle Herstellung stark ein.

Es ist weiterhin bekannt. Perfluoralkylsilylverbindungen durch Umsetzung von Perfluoralkylbromiden und Trialkylchlorsilanen in N-Methylpyrrolidon in Gegenwart von Aluminium herzustellen (Grobe et al., Synlett, 1995, 641-642). Auch bei diesem Verfahren sind die hohen Kosten der Ausgangsverbindungen nachteilig.

Langkettige Perfluoralkylsilane können durch Umsetzung von Perfluoralkylhalogenen und Trimethylchlorsilan über eine Grignard-Reaktion hergestellt werden (siehe beispielsweise Smith et al., J. Organomet. Chem. 46, 1972, 251-254). Nachteilig bei diesem Verfahren ist jedoch, dass die Methode sich nicht zur Herstellung kurzer Perfluoralkylsilane wie beispielsweise Trimethyltrifluormethylsilan eignet. Weiterhin nachteilig ist, dass sich Grignard-Reaktionen meist nur schwer kontrollieren lassen und sie daher für eine großtechnische Anwendung nur bedingt in Frage kommen.

Es bestand daher die Aufgabe, ein Verfahren bereitzustellen, welches eine technisch einfache Herstellung von Perfluoralkylsilanen ausgehend von preiswerten Ausgangsmaterialien erlaubt.

Überraschenderweise wurde nun ein Verfahren zur Herstellung von Polyfluoralkylsilanen der Formel (I) gefunden,

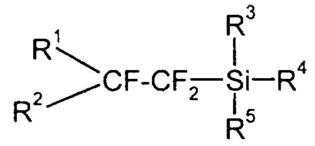

(I)

worin

$R^1$ und $R^2$ gleich oder verschieden sind und für Wasserstoff, Halogen, geradkettiges oder verzweigtes $C_1$-$C_8$-Alkyl, $C_3$-$C_9$-Cycloalkyl, $C_1$-$C_8$-Alkyloxy, $C_1$-$C_8$-Alkylthio, $C_6$-$C_{14}$-Aryl, durch $C_1$-$C_8$-Alkyl, Acyl, Sulfonyl oder $C_6$-$C_{14}$-Aryl disubstituiertes Amin, $C_6$-$C_{14}$-Aryloxy, $C_6$-$C_{14}$-Arylthio, Heteroaryl, Heteroaryloxy oder Cyano stehen, wobei die genannten Reste, außer Wasserstoff und Halogen, gegebenenfalls einen oder mehrere Substituenten aus der Reihe Halogen, $C_1$-$C_4$-Alkyl, $C_1$-$C_4$-Alkoxy, $C_6$-$C_{14}$-Aryloxy, Silyloxy, Nitro, Cyano, durch $C_1$-$C_4$-Alkyl, Acyl, Sulfonyl oder $C_6$-$C_{14}$-Aryl disubstituiertes Amin, Disilylamino und COO($C_1$-$C_6$-Alkyl) tragen,

$R^3$ bis $R^5$ gleich oder verschieden sind und für geradkettiges oder verzweigtes $C_1$-$C_8$-Alkyl oder $C_6$-$C_{14}$-Aryl stehen, welche gegebenenfalls einen oder mehrere Substituenten aus der Reihe Halogen, $C_1$-$C_4$-Alkyl, $C_1$-$C_4$-Alkoxy, $C_6$-$C_{14}$-Aryloxy, Silyloxy, Nitro, Cyano, durch $C_1$-$C_4$-Alkyl, Acyl, Sulfonyl oder $C_6$-$C_{14}$-Aryl disubstituiertes Amin, Disilylamino und COO($C_1$-$C_6$-Alkyl) tragen,

welches dadurch gekennzeichnet ist, dass Verbindungen der Formel (II)

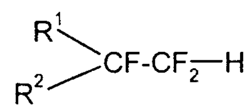

(II)

in der $R^1$ und $R^2$ die oben angegebene Bedeutung haben, mit Verbindungen der Formel (III)

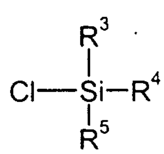

(III)

in der $R^3$ bis $R^5$ die oben angegebene Bedeutung haben, in Gegenwart einer Base umgesetzt werden.

In das erfindungsgemäße Verfahren werden vorzugsweise Verbindungen der Formel (II) eingesetzt, worin $R^1$ die oben genannte Bedeutung hat und $R^2$ für Halogen steht.

Besonders bevorzugt werden Verbindungen der Formel (II) eingesetzt, worin $R^1$ für Halogen, $C_1$-$C_8$-Alkyl, $C_1$-$C_8$-Alkoxy oder $C_6$-$C_{14}$-Aryloxy steht und $R^2$ für Fluor steht.

In das erfindungsgemäße Verfahren werden vorzugsweise Verbindungen der Formel (III) eingesetzt, worin $R^3$ bis $R^5$ gleich oder verschieden sind und für geradkettiges oder verzweigtes $C_1$-$C_6$-Alkyl stehen, welches gegebenenfalls einen oder mehrere Substituenten aus der Reihe Halogen, $C_1$-$C_4$-Alkyl, $C_1$-$C_4$-Alkoxy, $C_6$-$C_{14}$-Aryloxy, Silyloxy, Nitro, Cyano, durch $C_1$-$C_4$-Alkyl, Acyl, Sulfonyl oder $C_6$-$C_{14}$-Aryl disubstituiertes Amin, Disilylamino und COO($C_1$-$C_6$-Alkyl) trägt.

Besonders bevorzugt werden Verbindungen der Formel (III) eingesetzt, worin $R^3$ bis $R^5$ gleich sind und für $C_1$-$C_6$-Alkyl stehen.

Ganz besonders bevorzugt werden in das erfindungsgemäße Verfahren Verbindungen der Formel (II) eingesetzt, worin $R^1$ für Halogen, $C_1$-$C_8$-Alkyl, $C_1$-$C_8$-Alkoxy oder $C_6$-$C_{14}$-Aryloxy und $R^2$ für Fluor stehen und Verbindungen der Formel (III), worin $R^3$ bis $R^5$ gleich sind und für $C_1$-$C_6$-Alkyl stehen.

In das erfindungsgemäße Verfahren werden Verbindungen der Formel (III) vorzugsweise in einer Menge von 0,5 bis 20 Äquivalenten, insbesondere von 0,9 bis 5 Äquivalenten und ganz besonders bevorzugt von 1 bis 2 Äquivalenten, bezogen auf die Verbindungen der Formel (II), eingesetzt.

In das erfindungsgemäße Verfahren werden vorzugsweise starke Basen eingesetzt. Bevorzugte starke Basen sind beispielsweise starke anorganische Basen wie Natrium- und Kaliumhydrid. Diese werden vorzugsweise in Gegenwart eines Sulfons wie Tetramethylsulfon eingesetzt. Bevorzugte organische Basen sind Alkoxylate von Lithium, Natrium und Kalium wie Kalium-tert.-butoxylat, Alkyllithium-, Alkylnatrium- und Alkylkaliumverbindungen wie beispielsweise n-Butyllithium, Lithium- und Kaliumdialkylamide sowie Tris(trialkyl)silylamine. Besonders bevorzugte Basen sind n-Butyllithium, Kalium-tert.-butoxylat und Natriumhydrid in Gegenwart von Tetramethylsulfon. Die genannten Basen können gegebenenfalls durch Zugabe von Komplexbildnern wie beispielsweise Tetrakis-(dialkylamino)ethan aktiviert werden.

Es können auch Mischungen verschiedener starker Basen eingesetzt werden.

Die Base wird vorzugsweise in einer Menge von 0,5 bis 20 Äquivalenten, insbesondere von 0,9 bis 5 Äquivalenten und ganz besonders bevorzugt von 1 bis 2 Äquivalenten, bezogen auf die Verbindungen der Formel (II), eingesetzt.

Das erfindungsgemäße Verfahren kann in Substanz oder in Gegenwart eines Lösungsmittels durchgeführt werden. Wird das erfindungsgemäße Verfahren in Substanz durchgeführt, ist ein Überschuss an Verbindungen der Formel (III) vorteilhaft. Wird das erfindungsgemäße Verfahren in Gegenwart eines Lösungsmittels durchgeführt, so werden bevorzugt aprotische Lösungsmittel eingesetzt. Bevorzugte Lösungsmittel sind Mono- und Polyether, wie Tetrahydrofuran, Dialkylether oder Diglyme, Alkane wie Hexan oder Cyclohexan oder Aromaten wie Toluol oder Xylol. Besonders bevorzugte Lösungsmittel sind Methyl-tert.-butylether und Diethylether.

Die Umsetzung kann beispielsweise bei Temperaturen von -100°C bis +200°C, bevorzugt von -20°C bis +20°C, besonders bevorzugt von -5°C bis +10°C durchgeführt werden.

Die Umsetzung erfolgt vorzugsweise unter einem Schutzgas wie Stickstoff, Helium oder Argon.

Das erfindungsgemäße Verfahren kann so durchgeführt werden, dass Trialkylchlorsilan der Formel (III), gegebenenfalls in Anwesenheit eines Lösungsmittels, in einem Reaktionsgefäß vorgelegt wird. Die Verbindung der Formel (II) wird vorzugsweise zudosiert. Vorzugsweise erfolgt anschließend eine Zudosierung der Base. Die Mischung wird vorzugsweise gerührt, der Reaktionsfortschritt kann mittels gaschromatographischer Analyse kontrolliert werden. Die Aufarbeitung des Reaktionsgemisches kann destillativ erfolgen.

Die vorliegende Erfindung betrifft weiterhin Verbindungen der Formel (I), worin

$R^1$ und $R^2$ gleich oder verschieden sind und für Wasserstoff, Halogen, geradkettiges oder verzweigtes $C_1$-$C_8$-Alkyl, $C_3$-$C_9$-Cycloalkyl, $C_1$-$C_8$-Alkyloxy, $C_1$-$C_8$-Alkylthio, $C_6$-$C_{14}$-Aryl, durch $C_1$-$C_8$-Alkyl, Acyl, Sulfonyl oder $C_6$-$C_{14}$-Aryl disubstituiertes Amin, $C_6$-$C_{14}$-Aryloxy, $C_6$-$C_{14}$-Arylthio, Heteroaryl, Heteroaryloxy oder Cyano stehen, wobei die genannten Reste, außer Wasserstoff und Halogen, gegebenenfalls einen oder mehrere Substituenten aus der Reihe Halogen, $C_1$-$C_4$-Alkyl, $C_1$-$C_4$-Alkoxy, $C_6$-$C_{14}$-Aryloxy, Silyloxy, Nitro, Cyano, durch $C_1$-$C_4$-Alkyl, Acyl, Sulfonyl oder $C_6$-$C_{14}$-Aryl disubstituiertes Amin, Disilylamino und COO($C_1$-$C_6$-Alkyl) tragen,

$R^3$ bis $R^5$ gleich oder verschieden sind und für geradkettiges oder verzweigtes $C_1$-$C_8$-Alkyl oder $C_6$-$C_{14}$-Aryl stehen, welche gegebenenfalls einen oder mehrere Substituenten aus der Reihe Halogen, $C_1$-$C_4$-Alkyl, $C_1$-$C_4$-Alkoxy, $C_6$-$C_{14}$-Aryloxy, Silyloxy, Nitro, Cyano, durch $C_1$-$C_4$-Alkyl, Acyl, Sulfonyl oder $C_6$-$C_{14}$-Aryl disubstituiertes Amin, Disilylamino und COO($C_1$-$C_6$-Alkyl) tragen,

mit der Maßgabe, dass $R^1$ nicht Wasserstoff, Halogen oder Fluoralkyl ist, wenn $R^2$ Fluor oder Chlor ist.

Bevorzugte Verbindungen der Formel (1) sind solche, worin $R^1$ die oben genannte Bedeutung hat, $R^2$ für Halogen steht und $R^3$ bis $R^5$ gleich oder verschieden sind und für geradkettiges oder verzweigtes $C_1$-$C_6$-Alkyl stehen, welches gegebenenfalls einen oder mehrere Substituenten aus der Reihe Halogen, $C_1$-$C_4$-Alkyl, $C_1$-$C_4$-Alkoxy, $C_6$-$C_{14}$-Aryloxy, Silyloxy, Nitro, Cyano, durch $C_1$-$C_4$-Alkyl, Acyl, Sulfonyl oder $C_6$-$C_{14}$-Aryl disubstituiertes Amin, Disilylamino und COO($C_1$-$C_6$-Alkyl) trägt.

Besonders bevorzugte Verbindungen der Formel (I) sind solche, worin $R^1$ für Halogen, $C_1$-$C_8$-Alkyl, $C_1$-$C_8$-Alkoxy oder $C_6$-$C_{14}$-Aryloxy steht, $R^2$ für Fluor steht und $R^3$ bis $R^5$ gleich sind und für $C_1$-$C_6$-Alkyl stehen.

Die erfindungsgemäßen Polyfluoralkylsilane sowie die nach dem erfindungsgemäßen Verfahren hergestellten Polyfluoralkylsilane können als Überträger von Polyfluoralkylgruppen in der synthetischen Chemie zur Herstellung von Wirkstoffen im Bereich der Pharmazie oder Agrochemie dienen. Darüberhinaus können die erfindungsgemäßen Polyfluoralkylsilane sowie die nach dem erfindungsgemäßen Verfahren hergestellten Polyfluoralkylsilane zur Herstellung von hochwertigen Flüssigkristallen für optische Anzeigen verwendet werden. Damit wird die Einführung neuer Polyfluoralkylgruppen in potentielle Wirkstoffe und Flüssigkristalle möglich. Zudem können die Kosten für die Herstellung von polyfluoralkylierten Verbindungen durch die niedrigen Herstellkosten der erfindungsgemäßen Polyfluoralkylsilane gesenkt werden.

Die vorliegende Erfindung betrifft weiterhin Flüssigkristalle der Formel (IV)

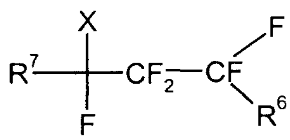

(IV)

worin

X für Fluor oder Wasserstoff steht,

$R^6$ für Wasserstoff, Fluor, Polyfluor-$C_1$-$C_8$-alkyl, $C_1$-$C_8$-Alkoxy, $C_6$-$C_{14}$-Aryloxy oder Heteroaryloxy steht, wobei die genannten Reste, außer Wasserstoff und Fluor, gegebenenfalls einen oder mehrere Substituenten aus der Reihe Halogen, $C_1$-$C_4$-Alkyl, $C_1$-$C_4$-Alkoxy, $C_6$-$C_{14}$-Aryloxy, Silyloxy, Nitro, Cyano, durch $C_1$-$C_4$-Alkyl, Acyl, Sulfonyl oder $C_6$-$C_{14}$-Aryl disubstituiertes Amin, Disilylamino und COO($C_1$-$C_6$-Alkyl) tragen und

$R^7$ für gegebenenfalls substituiertes $C_6$-$C_{14}$-Aryl oder $C_6$-$C_{18}$-Cycloalkyl steht,

Bevorzugte Flüssigkristalle der Formel (IV) sind solche, worin

X für Wasserstoff steht,

$R^6$ für Wasserstoff, Fluor, Polyfluor-$C_1$-$C_4$-Alkyl, geradkettiges oder verzweigtes $C_1$-$C_4$-Alkoxy steht, welche außer Wasserstoff, Fluor und Polyfluoralkyl, durch ein oder mehrere Fluoratome substituiert sind, und

$R^7$ für einen Phenylrest, einen Biphenylrest oder einen Cyclohexylrest steht, welche gegebenenfalls einen oder mehrere Substituenten aus der Reihe geradkettiges oder verzweigtes $C_4$-$C_{18}$-Alkyl, $C_1$-$C_{12}$-Alkyl-$C_5$-$C_8$-CycloalkylundC$_5$-$C_8$-Cycloalkyl tragen.

Besonders bevorzugte Flüssigkristalle der Formel (IV) sind solche, worin

X für Wasserstoff,

$R^6$ für Wasserstoff, Fluor, Trifluormethyl oder Pentafluorethyl und

$R^7$ für einen Phenylrest stehen, welcher in para-Stellung einen Substituenten aus der Reihe $C_4$-$C_{18}$-Alkyl und $C_1$-$C_{12}$-Alkyl-$C_5$-$C_8$-Cycloalkyl trägt.

Die vorliegende Erfindung betrifft weiterhin ein Verfahren zur Herstellung von Flüssigkristallen der Formel (IV) durch Umsetzung von Verbindungen der Formel (I),

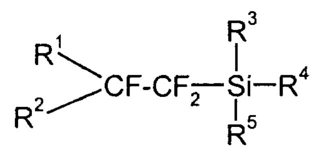

(I)

worin

$R^1$ den gleichen Bedeutungsumfang hat wie $R^6$ bei Formel (IV),

$R^2$ für Fluor steht und

$R^3$ bis $R^5$ gleich oder verschieden sind und für geradkettiges oder verzweigtes $C_1$-$C_8$-Alkyl oder $C_6$-$C_{14}$-Aryl stehen, welche gegebenenfalls einen oder mehrere Substituenten aus der Reihe Halogen, $C_1$-$C_4$-Alkyl, $C_1$-$C_4$-Alkoxy, $C_6$-$C_{14}$-Aryloxy, Silyloxy, Nitro, Cyano, durch $C_1$-$C_4$-Alkyl, Acyl, Sulfo-

nyl oder $C_6$-$C_{14}$-Aryl disubstituiertes Amin, Disilylamino und COO($C_1$-$C_6$-Alkyl) tragen,

mit Verbindungen der Formel (V)

$$R^7\text{-}Y \quad (V)$$

worin

Y für eine gegenüber -$SiR^3R^4R^5$ reaktive Gruppe steht, vorzugsweise für eine Aldehyd-, Keto- oder Estergruppe, und

$R^7$ für gegebenenfalls substituiertes $C_6$-$C_{14}$-Aryl oder $C_6$-$C_{18}$-Cycloalkyl steht,

in Gegenwart von Verbindungen, die eine starke Affinität zu Silizium aufweisen, so Verbindungen der Formel (VI) erhält

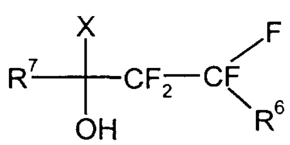

(VI),

in der

$R^6$ den gleichen Bedeutungsumfang hat wie $R^1$ in Formel (I) und

$R^7$ die bei Formel (V) angegebene Bedeutung hat,

und diese anschließend mit Fluorierungsmitteln umsetzt.

Verbindungen, die eine starke Affinität zu Silizium aufweisen sind beispielsweise solche, die als Fluorid- oder Alkoxyspender fungieren, beispielsweise Fluoride wie Tetrabutylammoniumfluorid oder Alkoxylate wie Natriummethanolat.

Bevorzugte Fluoride sind Alkylammoniumfluoride wie beispielsweise Tetrabutylammoniumfluorid oder Alkalifluoride wie beispielsweise Natriumfluorid oder Kaliumfluorid. Besonders bevorzugt ist Tetrabutylammoniumfluorid.

Bevorzugte Alkoxylate sind Alkalialkoholate wie beispielsweise Natriummethanolat oder Natriumethanolat.

Fluorierungsmittel sind Verbindungen, die in der Lage sind in Verbindungen der Formel (VI) OH gegen F auszutauschen wie beispielsweise Diethylaminoschwefeltrifluorid.

Vorzugsweise werden im erfindungsgemäßen Verfahren zur Herstellung von Verbindungen der Formel (IV) Verbindungen der Formel (I) eingesetzt, worin

$R^1$ den bevorzugten Bedeutungsumfang hat wie für $R^6$ bei Formel (IV) angegeben,

$R^2$ für Fluor steht und

$R^3$ bis $R^5$ gleich oder verschieden sind und für geradkettiges oder verzweigtes $C_1$-$C_6$-Alkyl oder $C_6$-$C_{14}$-Aryl stehen, welche gegebenenfalls einen oder mehrere Substituenten aus der Reihe Halogen, $C_1$-$C_4$-Alkyl, $C_1$-$C_4$-Alkoxy, $C_6$-$C_{14}$-Aryloxy, Silyloxy, Nitro, Cyano, durch $C_1$-$C_4$-Alkyl, Acyl, Sulfonyl oder $C_6$-$C_{14}$-Aryl disubstituiertes Amin, Disilylamino und COO($C_1$-$C_6$-Alkyl) tragen.

Besonders bevorzugt werden im erfindungsgemäßen Verfahren zur Herstellung von Verbindungen der Formel (IV) Verbindungen der Formel (I) eingesetzt, worin

$R^1$ Wasserstoff, Fluor, Trifluormethyl oder Pentafluorethyl und

$R^2$ für Fluor stehen und

$R^3$ bis $R^5$ gleich sind und für $C_1$-$C_6$-Alkyl stehen.

Vorzugsweise werden im erfindungsgemäßen Verfahren zur Herstellung von Verbindungen der Formel (IV) Verbindungen der Formel (V) eingesetzt, worin

$R^7$ für einen Phenylrest, einen Biphenylrest oder einen $C_6$-$C_{18}$-Cyclohexylrest steht, welche gegebenenfalls Substituenten aus der Reihe geradkettiges oder verzweigtes $C_4$-$C_{18}$-Alkyl, $C_1$-$C_{12}$-Alkyl-$C_5$-$C_8$-cycloalkyl und $C_5$-$C_8$-Cycloalkyl tragen und

Y für einen Aldehyd-, Keto- oder Estergruppe steht.

Besonders bevorzugt werden Verbindungen der Formel (V) eingesetzt, worin

$R^7$ für einen Phenylrest steht, welcher in para-Stellung einen Substituenten aus der Reihe $C_4$-$C_{18}$-Alkyl und $C_1$-$C_{12}$-Alkyl-$C_5$-$C_8$-cycloalkyl trägt und

Y für eine Aldehyd-, Keto- oder Estergruppe steht.

In einer besonders bevorzugten Ausführungsform werden im erfindungsgemäßen Verfahren zur Herstellung von Verbindungen der Formel (IV) Verbindungen der Formel (I), worin

$R^1$ für Wasserstoff, Fluor, Trifluormethyl oder

Pentafluorethyl,

$R^2$ für Fluor und

$R^3$ bis $R^5$ gleich sind und für $C_1$-$C_6$-Alkyl stehen,

mit Verbindungen der Formel (V), worin

$R^7$ für einen Phenyl- oder Biphenylrest steht, welcher in para-Stellung einen Substituenten aus der Reihe $C_4$-$C_{18}$-Alkyl- und $C_1$-$C_{12}$-Alkyl-$C_5$-$C_8$-cyclohexyl trägt und

Y für eine Aldehyd-, Keto- oder Estergruppe steht

in Gegenwart von Fluoriden oder Alkoxylaten zu Verbindungen der Formel (VI)

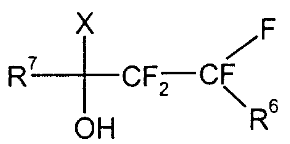
(VI)

umgesetzt und diese mit dem Fluorierungsmittel Diethylaminoschwefeltrifluorid zu Verbindungen der Formel (IV) umgesetzt.

Im erfindungsgemäßen Verfahren zur Herstellung von Verbindungen der Formel (IV) werden vorzugsweise 0,5 bis 20 Äquivalente, insbesondere 0,9 bis 5 Äquivalente, ganz besonders bevorzugt 1 bis 2 Äquivalente an Verbindungen der Formel (I), bezogen auf die Verbindungen der Formel (V) eingesetzt.

## Beispiele

### Beispiel 1

In einem 1 l Vierhalskolben mit Rührer, Thermometer und Gaseinlasskapillare wurden unter Stickstoff 300 ml Methyl-tert.-butylether und 13,6 g (125,1 mmol) Trimethylchlorsilan vorgelegt und auf 0°C abgekühlt. Nach Erreichen der Zieltemperatur wurden 15,75 g (81,2 mmol) Phenoxytetrafluorethan zugetropft. Anschließend wurden unter verstärkter Kühlung 50 ml (125 mmol) n-Butyllithium langsam zugetropft. Das Gemisch wurde bei 0°C gerührt und der Reaktionsfortschritt mittels Gaschromatographie kontrolliert. Nach Beendigung der Reaktion wurde die trübe Lösung filtriert und das Filtrat destilliert.
Ausbeute: 9,2 g 1,1,2,2,-Tetrafluorethyl-2-phenoxy-trimethylsilan (43 % der Theorie).
Siedepunkt: 91 bis 92,5°C bei 10 mbar.
Massenspektrometrie (m/z): ($M^{+\cdot}$)266, ($M^{+\cdot}$-115) 151, ($M^{+\cdot}$-189)77, ($M^{+\cdot}$-215)51 $^{19}$F-NMR-Daten δ: -86ppm (s, 2F, $CF_2$); -131ppm (s, 2F, $CF_2$)

### Beispiel 2

In einem 1l Vierhalskolben mit Rührer, Thermometer und Gaseinlasskapillare wurden unter Stickstoff 300 ml Methyl-tert.-butylether und 12,5 g (150 mmol) Triethylchlorsilan vorgelegt und auf 0°C abgekühlt. Nach Erreichen der Zieltemperatur wurden 10 g (83,4 mmol) Pentafluorethan zugetropft. Anschließend wurden unter verstärkter Kühlung 62,5 ml (125 mmol) n-Butyllithium langsam zugetropft. Das Gemisch wurde bei 0°C gerührt und der Reaktionsfortschritt mittels Gaschromatographie kontrolliert. Nach Beendigung der Reaktion wurde die trübe Lösung filtriert und das Filtrat destilliert.
Ausbeute: 10.5 g Triethyl-pentafluorethylsilan (54 % der Theorie).
Siedepunkt: 50°C bei 30 mbar

### Beispiel 3

In einem 1l Vierhalskolben mit Rührer, Thermometer und Gaseinlasskapillare wurden unter Stickstoff 450 ml Methyl-tert.-butylether und 27g (180 mmol) Triethylchlorsilan vorgelegt und auf 0°C abgekühlt. Nach Erreichen der Zieltemperatur wurden 23,8 g (180 mmol) Methoxy-tetrafluorethan zugetropft. Anschließend wurden unter verstärkter Kühlung 135 ml (270 mmol) n-Butyllithium langsam zugetropft. Das Gemisch wurde bei 0°C gerührt und der Reaktionsfortschritt mittels Gaschromatographie kontrolliert. Nach Beendigung der Reaktion wurde die trübe Lösung filtriert, das Filtrat destilliert und 1,1,2,2,-Tetrafluorethyl-2-methoxytriethylsilan als 10 %ige Lösung in Butyltriethylsilan erhalten
Siedepunkt: 32°C bei 64 mbar.

Massenspektrometrie (m/z): ($M^{+\cdot}$-29)217, ($M^{+\cdot}$-129) 127, ($M^{+\cdot}$-153)77, ($M^{+\cdot}$-187)59, ($M^{+\cdot}$-217)29 $^{19}$F-NMR-Daten δ: -93ppm (s, 2F, $CF_2$); -126ppm (s, 2F, $CF_2$)

## Patentansprüche

1. Verfahren zur Herstellung von Verbindungen der Formel (I)

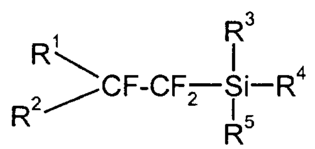
(I)

worin

$R^1$ und $R^2$ gleich oder verschieden sind und für Wasserstoff, Halogen, geradkettiges oder verzweigtes $C_1$-$C_8$-Alkyl,

$C_3$-$C_9$-Cycloalkyl, $C_1$-$C_8$-Alkyloxy, $C_1$-$C_8$-Alkylthio, $C_6$-$C_{14}$-Aryl, durch $C_1$-$C_8$-Alkyl, Acyl, Sulfonyl oder $C_6$-$C_{14}$-Aryl disubstituiertes Amin, $C_6$-$C_{14}$-Aryloxy, $C_6$-$C_{14}$-Arylthio, Heteroaryl, Heteroaryloxy oder Cyano stehen, wobei die genannten Reste, außer Wasserstoff und Halogen, gegebenenfalls einen oder mehrere Substituenten aus der Reihe Halogen, $C_1$-$C_4$-Alkyl, $C_1$-$C_4$-Alkoxy, $C_6$-$C_{14}$-Aryloxy, Silyloxy, Nitro, Cyano, durch $C_1$-$C_4$-Alkyl, Acyl, Sulfonyl oder $C_6$-$C_{14}$-Aryl disubstituiertes Amin, Disilylamino und COO($C_1$-$C_6$-Alkyl) tragen,

$R^3$ bis $R^5$ gleich oder verschieden sind und für geradkettiges oder verzweigtes $C_1$-$C_8$-Alkyl oder $C_6$-$C_{14}$-Aryl stehen, welche gegebenenfalls einen oder mehrere Substituenten aus der Reihe Halogen, $C_1$-$C_4$-Alkyl, $C_1$-$C_4$-Alkoxy, $C_6$-$C_{14}$-Aryloxy, Silyloxy, Nitro, Cyano, durch $C_1$-$C_4$-Alkyl, Acyl, Sulfonyl oder $C_6$-$C_{14}$-Aryl disubstituiertes Amin, Disilylamino und COO($C_1$-$C_6$-Alkyl) tragen,

**dadurch gekennzeichnet, dass** Verbindungen der Formel (II)

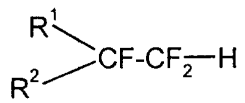

(II)

in der $R^1$ und $R^2$ die oben angegebene Bedeutung haben,
mit Verbindungen der Formel (III)

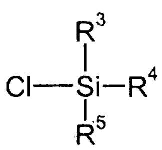

(III)

in der $R^3$ bis $R^5$ die oben angegebene Bedeutung haben,
in Gegenwart einer Base umgesetzt werden.

**2.** Verfahren gemäß Anspruch 1, **dadurch gekennzeichnet, dass** Verbindungen der Formel (II) eingesetzt werden, worin

$R^1$ die im Anspruch 1 angegebene Bedeutung hat und

$R^2$ für Halogen steht.

**3.** Verfahren gemäß Ansprüchen 1 bis 2, **dadurch gekennzeichnet, dass** Verbindungen der Formel (III) eingesetzt werden, worin

$R^3$ bis $R^5$ gleich oder verschieden sind und für geradkettiges oder verzweigtes $C_1$-$C_6$-Alkyl stehen, welches gegebenenfalls einen oder mehrere Substituenten aus der Reihe Halogen, $C_1$-$C_4$-Alkyl, $C_1$-$C_4$-Alkoxy, $C_6$-$C_{14}$-Aryloxy, Silyloxy, Nitro, Cyano, durch $C_1$-$C_4$-Alkyl, Acyl, Sulfonyl oder $C_6$-$C_{14}$-Aryl disubstituiertes Amin, Disilylamino und COO($C_1$-$C_6$-Alkyl) trägt.

**4.** Verfahren gemäß Ansprüchen 1 bis 3, **dadurch gekennzeichnet, dass** es sich bei der Base um eine starke Base handelt.

**5.** Verfahren gemäß Ansprüchen 1 bis 4, **dadurch gekennzeichnet, dass** die Base in einer Menge von 0,5 bis 20 Äquivalenten, bezogen auf die Verbindungen der Formel (II), eingesetzt wird.

**6.** Verbindungen der Formel (I)

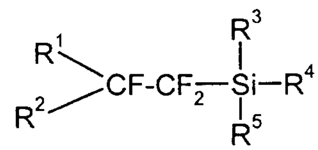

(I)

worin

$R^1$ und $R^2$ gleich oder verschieden sind und für Wasserstoff, Halogen, geradkettiges oder verzweigtes $C_1$-$C_8$-Alkyl, $C_3$-$C_9$-Cycloalkyl, $C_1$-$C_8$-Alkyloxy, $C_1$-$C_8$-Alkylthio, $C_6$-$C_{14}$-Aryl, durch $C_1$-$C_8$-Alkyl, Acyl, Sulfonyl oder $C_6$-$C_{14}$-Aryl disubstituiertes Amin, $C_6$-$C_{14}$-Aryloxy, $C_6$-$C_{14}$-Arylthio, Heteroaryl, Heteroaryloxy oder Cyano stehen, wobei die genannten Reste, außer Wasserstoff und Halogen, gegebenenfalls einen oder mehrere Substituenten aus der Reihe Halogen, $C_1$-$C_4$-Alkyl, $C_1$-$C_4$-Alkoxy, $C_6$-$C_{14}$-Aryloxy, Silyloxy, Nitro, Cyano, durch $C_1$-$C_4$-Alkyl, Acyl, Sulfonyl oder $C_6$-$C_{14}$-Aryl disubstituiertes Amin, Disilylamino und COO($C_1$-$C_6$-Alkyl) tragen,

$R^3$ bis $R^5$ gleich oder verschieden sind und für geradkettiges oder verzweigtes $C_1$-$C_8$-Alkyl oder $C_6$-$C_{14}$-Aryl stehen, welche gegebenenfalls einen oder mehrere Substituenten aus der Reihe Halogen, $C_1$-$C_4$-Alkyl, $C_1$-$C_4$-Alkoxy, $C_6$-$C_{14}$-Aryloxy, Silyloxy, Nitro, Cyano, durch $C_1$-$C_4$-Alkyl, Acyl, Sulfonyl oder $C_6$-$C_{14}$-Aryl disubstituiertes Amin, Disilylamino und COO($C_1$-$C_6$-Alkyl) tragen,

mit der Maßgabe, dass $R^1$ nicht Wasserstoff, Halogen oder Fluoralkyl ist, wenn $R^2$ Fluor oder Chlor ist.

**7.** Verbindungen gemäß Anspruch 6, worin

$R^1$ die im Anspruch 6 angegebene Bedeutung hat,

$R^2$ für Halogen steht und

$R^3$ bis $R^5$ gleich oder verschieden sind und für geradkettiges oder verzweigtes $C_1$-$C_6$-Alkyl stehen, welches gegebenenfalls einen oder mehrere Substituenten aus der Reihe Halogen, $C_1$-$C_4$-Alkyl, $C_1$-$C_4$-Alkoxy, $C_6$-$C_{14}$-Aryloxy, Silyloxy, Nitro, Cyano, durch $C_1$-$C_4$-Alkyl, Acyl, Sulfonyl oder $C_6$-$C_{14}$-Aryl disubstituiertes Amin, Disilylamino und COO($C_1$-$C_6$-Alkyl) trägt,

mit der Maßgabe, dass $R^1$ nicht Wasserstoff, Halogen oder Fluoralkyl ist, wenn $R^2$ Fluor oder Chlor ist.

**8.** Verwendung der Verbindungen der Formel (I) gemäß Anspruch 1 zur Herstellung von Pharmazeutika und Agrochemikalien.

**9.** Verwendung der Verbindungen der Formel (I) gemäß Anspruch 1 zur Herstellung von Flüssigkristallen.

**10.** Flüssigkristalle der Formel (IV)

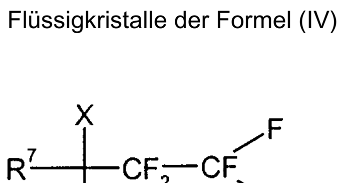

(IV)

worin

X für Fluor oder Wasserstoff steht,

$R^6$ für Wasserstoff, Fluor, Polyfluor-$C_1$-$C_8$-alkyl, $C_1$-$C_4$-Alkoxy, $C_6$-$C_{14}$-Aryloxy oder Heteroaryloxy steht, wobei die genannten Reste, außer Wasserstoff und Fluor, gegebenenfalls einen oder mehrere Substituenten aus der Reihe Halogen, $C_1$-$C_4$-Alkyl, $C_1$-$C_4$-Alkoxy, $C_6$-$C_{14}$-Aryloxy, Silyloxy, Nitro, Cyano, durch $C_1$-$C_4$-Alkyl. Acyl, Sulfonyl oder $C_6$-$C_{14}$-Aryl disubstituiertes Amin, Disilylamino und COO ($C_1$-$C_6$-Alkyl) tragen und

$R^7$ für gegebenenfalls substituiertes $C_6$-$C_{14}$-Aryl oder $C_6$-$C_{18}$-Cycloalkyl steht.

**11.** Flüssigkristalle gemäß Anspruch 10, **dadurch gekennzeichnet, dass**

X für Wasserstoff steht,

$R^6$ für Wasserstoff, Fluor, Polyfluor-$C_1$-$C_4$-Alkyl, geradkettiges oder verzweigtes $C_1$-$C_8$-Alkoxy, welche, außer Wasserstoff, Fluor und Perfluoralkyl durch ein oder mehrere Fluoratome substituiert sind, steht und

$R^7$ für einen Phenylrest, einen Biphenylrest oder einen Cyclohexylrest steht, welche gegebenenfalls einen oder mehrere Substituenten aus der Reihe geradkettiges oder verzweigtes$C_4$-$C_{18}$-Alkyl,$C_1$-$C_{12}$-Alkyl-$C_5$-$C_8$-Cycloalkyl und $C_5$-$C_8$-Cycloalkyl tragen.

**12.** Verfahren zur Herstellung von Flüssigkristallen gemäß Anspruch 1 durch Umsetzung von Verbindungen der Formel (I),

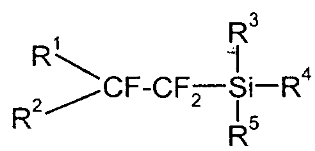

(I)

worin

$R^1$ den gleichen Bedeutungsumfang hat wie $R^6$ bei Formel (IV) in Anspruch 10,

$R^2$ für Fluor steht und

$R^3$ bis $R^5$ gleich oder verschieden sind und für geradkettiges oder verzweigtes $C_1$-$C_8$-Alkyl oder $C_6$-$C_{14}$-Aryl stehen, welche gegebenenfalls einen oder mehrere Substituenten aus der Reihe Halogen, $C_1$-$C_4$-Alkyl, $C_1$-$C_4$-Alkoxy, $C_6$-$C_{14}$-Aryloxy, Silyloxy, Nitro, Cyano, durch $C_1$-$C_4$-Alkyl, Acyl, Sulfonyl oder $C_6$-$C_{14}$-Aryl disubstituiertes Amin, Di-

silylamino und $COO(C_1-C_6\text{-Alkyl})$ tragen,

mit Verbindungen der Formel (V)

$$R^7\text{-}Y \quad (V)$$

worin

Y für eine gegenüber $-SiR^3R^4R^5$ reaktive Gruppe und

$R^7$ für gegebenenfalls substituiertes $C_6$-$C_{14}$-Aryl oder $C_6$-$C_{18}$-Cycloalkyl steht,

in Gegenwart von Verbindungen, die eine starke Affinität zu Silizium aufweisen, so Verbindungen der Formel (VI) erhält

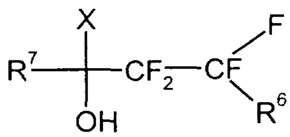

(VI)

in der

$R^6$ den gleichen Bedeutungsumfang hat wie $R^1$ in Formel (I) und

$R^7$ die bei Formel (V) angegebene Bedeutung hat,

und dies anschließend mit Fluorierungsmitteln umsetzt.

**13.** Verwendung der Flüssigkristalle gemäß Anspruch 10 für optische Anzeigen.

Europäisches Patentamt

# EUROPÄISCHER RECHERCHENBERICHT

Nummer der Anmeldung
EP 01 11 4733

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | DATABASE BEILSTEIN 'Online! BEILSTEIN INSTITUTE FOR ORGANIC CHEMISTRY, FRANKFURT/MAIN, DE; Database accession no. Product BRN 1985790 XP002175704 * Zusammenfassung * | 6,7 | C07F7/08 |
| A | Database accession no 3948495 (reaction ID) & CHEN, G. J. RET AL.: J. ORGANOMET. CHEM., Nr. 293, 1985, Seiten 313-322, --- | 1-5 | |
| X | DATABASE BEILSTEIN 'Online! BEILSTEIN INSTITUTE FOR ORGANIC CHEMISTRY, FRANKFURT/MAIN, DE; Database accession no. BRN 2271549 XP002175705 * Zusammenfassung * --- | 6 | |
| X | DATABASE CHEMABS 'Online! CHEMICAL ABSTRACTS SERVICE, COLUMBUS, OHIO, US; ZIMIN, A. V. ET AL: "Study of the characteristics of the radiation-induced-chemical hydrosilylation of halogenated and nonhalogenated olefins" retrieved from STN Database accession no. 86:121428 XP002175706 * Zusammenfassung * & DOKL. AKAD. NAUK SSSR (1976), 231(4), 870-3 'CHEM.! , --- | 6 | RECHERCHIERTE SACHGEBIETE (Int.Cl.7) C07F |

-/--

Der vorliegende Recherchenbericht wurde für alle Patentansprüche erstellt

| Recherchenort | Abschlußdatum der Recherche | Prüfer |
|---|---|---|
| MÜNCHEN | 24. August 2001 | Richter, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

Europäisches Patentamt

# EUROPÄISCHER RECHERCHENBERICHT

Nummer der Anmeldung
EP 01 11 4733

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | BARTMANN E: "FLUESSIGKRISTALLE MIT FLUORHALTIGEN ALKYLGRUPPEN" BERICHTE DER BUNSEN-GESELLSCHAFT FUR PHYSIKALISCHE CHEMIE, VERLAG CHEMIE. WEINHEIM, DE, Bd. 97, Nr. 10, 1993, Seiten 1349-1355, XP000891537 ISSN: 0005-9021 --- | 10,11,13 | |
| X | DATABASE BEILSTEIN 'Online! BEILSTEIN INSTITUTE FOR ORGANIC CHEMISTRY, FRANKFURT/MAIN, DE; Database accession no. BRN 5131085; BRN 5127207 XP002175708 * Zusammenfassung * & FIALKOV, YU. A. ET AL.: J.ORG.CHEM. USSR (ENGL. TRANSL.), Bd. 19, Nr. 5, 1983, Seiten 933-937, --- | 10,13 | |
| X | DATABASE BEILSTEIN 'Online! BEILSTEIN INSTITUTE FOR ORGANIC CHEMISTRY, FRANKFURT/MAIN, DE; Database accession no. BRN 5306502 XP002175709 * Zusammenfassung * & FIALKOV,YU. A. ET AL.: J.ORG.CHEM. USSR, Bd. 19, Nr. 10, 1983, Seiten 1781-1787, --- | 10,13 | RECHERCHIERTE SACHGEBIETE (Int.Cl.7) |
| X | DE 37 30 859 A (MERCK PATENT GMBH) 30. März 1989 (1989-03-30) * Beispiel 3 * --- | 10,13 | |
| X | US 4 886 620 A (HOPF, REINHARD ET AL) 12. Dezember 1989 (1989-12-12) * Beispiel 38 * --- -/-- | 10,13 | |

Der vorliegende Recherchenbericht wurde für alle Patentansprüche erstellt

| Recherchenort | Abschlußdatum der Recherche | Prüfer |
|---|---|---|
| MÜNCHEN | 24. August 2001 | Richter, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

Europäisches Patentamt

# EUROPÄISCHER RECHERCHENBERICHT

Nummer der Anmeldung
EP 01 11 4733

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | DATABASE CHEMABS 'Online!<br>CHEMICAL ABSTRACTS SERVICE, COLUMBUS, OHIO, US;<br>GORRIA, P. ET AL: "Influence of structure on smectic A-smectic A phase separation"<br>retrieved from STN<br>Database accession no. 125:261894<br>XP002175710<br>* Zusammenfassung *<br>& LIQ. CRYST. (1996), 21(4), 523-530 ,<br>--- | 10 | |
| X | DATABASE CHEMABS 'Online!<br>CHEMICAL ABSTRACTS SERVICE, COLUMBUS, OHIO, US;<br>YAMADA, NOBUTSUGU ET AL: "Liquid crystal element using chiral smectic liquid crystal"<br>retrieved from STN<br>Database accession no. 126:299740<br>XP002175711<br>* Zusammenfassung *<br>& JP 09 059628 A (CANON KK, JAPAN)<br>4. März 1997 (1997-03-04) | 10 | RECHERCHIERTE SACHGEBIETE (Int.Cl.7) |
| X | * Seite 9, Spalte 2, Zeile 5,9,10 * | 10 | |
| X | * Seite 10, Spalte 1, Zeile 5,9,10 * | 10 | |
| | * Seite 10, Spalte 2, Zeile 5,9,10 *<br>---<br>-/-- | | |

Der vorliegende Recherchenbericht wurde für alle Patentansprüche erstellt

| Recherchenort | Abschlußdatum der Recherche | Prüfer |
|---|---|---|
| MÜNCHEN | 24. August 2001 | Richter, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie,übereinstimmendes Dokument

EPO FORM 1503 03.82 (P04C03)

Europäisches Patentamt

# EUROPÄISCHER RECHERCHENBERICHT

Nummer der Anmeldung
EP 01 11 4733

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | DATABASE CHEMABS 'Online!<br>CHEMICAL ABSTRACTS SERVICE, COLUMBUS, OHIO, US;<br>HIYAMA, TAMEJIRO ET AL: "Preparation of 1-arylpolyhalopropanes"<br>retrieved from STN<br>Database accession no. 116:151299<br>XP002175712<br>* Zusammenfassung *<br>& JP 03 258736 A (SAGAMI CHEMICAL RESEARCH CENTER, JAPAN)<br>19. November 1991 (1991-11-19)<br>Reaktionsgleichung 8, Ausgangsmaterial<br>* Seite 9, Spalte 2 *<br>----- | 10 | |

RECHERCHIERTE SACHGEBIETE (Int.Cl.7)

Der vorliegende Recherchenbericht wurde für alle Patentansprüche erstellt

| Recherchenort | Abschlußdatum der Recherche | Prüfer |
|---|---|---|
| MÜNCHEN | 24. August 2001 | Richter, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 01 11 4733

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-08-2001

| Im Recherchenbericht angeführtes Patentdokument | Datum der Veröffentlichung | Mitglied(er) der Patentfamilie | Datum der Veröffentlichung |
|---|---|---|---|
| DE 3730859 A | 30-03-1989 | DE 3712995 A | 03-11-1988 |
| | | AT 82999 T | 15-12-1992 |
| | | DE 3876356 A | 14-01-1993 |
| | | DE 3876356 D | 14-01-1993 |
| | | WO 8808019 A | 20-10-1988 |
| | | EP 0309514 A | 05-04-1989 |
| | | JP 2500191 T | 25-01-1990 |
| | | US 5478496 A | 26-12-1995 |
| | | DD 281202 A | 01-08-1990 |
| US 4886620 A | 12-12-1989 | DE 3533333 A | 26-03-1987 |
| | | DE 3608500 A | 24-09-1987 |
| | | DD 252196 A | 09-12-1987 |
| | | DD 252197 A | 09-12-1987 |
| | | DE 3650014 D | 08-09-1994 |
| | | DE 3685213 A | 11-06-1992 |
| | | DE 3685213 D | 11-06-1992 |
| | | WO 8701717 A | 26-03-1987 |
| | | WO 8701701 A | 26-03-1987 |
| | | EP 0233267 A | 26-08-1987 |
| | | EP 0238576 A | 30-09-1987 |
| | | JP 2777885 B | 23-07-1998 |
| | | JP 63500948 T | 07-04-1988 |
| | | JP 63500945 T | 07-04-1988 |
| | | NO 872039 A | 15-05-1987 |
| | | NO 872040 A | 15-05-1987 |
| | | US 5002694 A | 26-03-1991 |
| | | US 5075032 A | 24-12-1991 |
| | | DD 249707 A | 16-09-1987 |
| | | DE 3670078 D | 10-05-1990 |
| | | WO 8705618 A | 24-09-1987 |
| | | EP 0259346 A | 16-03-1988 |
| | | JP 63502657 T | 06-10-1988 |
| JP 9059628 A | 04-03-1997 | KEINE | |
| JP 3258736 A | 19-11-1991 | KEINE | |

EPO FORM P0461

Für nähere Einzelheiten zu diesem Anhang : siehe Amtsblatt des Europäischen Patentamts, Nr.12/82